Europäisches Patentamt

European Patent Office

Office européen des brevets

(19)

(11) Publication number: **0 228 810**
**B1**

(12) **EUROPEAN PATENT SPECIFICATION**

(45) Date of publication of the patent specification:
**11.07.90**

(21) Application number: **86309181.5**

(22) Date of filing: **25.11.86**

(51) Int. Cl.⁵: **G01N 33/74**, G01N 33/577,
G01N 33/535

(54) **Enzyme immunoassay method for epidermal growth factor.**

(30) Priority: **25.11.85 JP 265822/85**

(43) Date of publication of application:
**15.07.87 Bulletin 87/29**

(45) Publication of the grant of the patent:
**11.07.90 Bulletin 90/28**

(84) Designated Contracting States:
**CH DE FR GB IT LI**

(56) References cited:
CHEMICAL ABSTRACTS, vol. 103, no. 7, Aug 19, 1985, Columbus, Ohio, USA; HAYASHI et al. "A sensitive twosite enzyme immunoassay for human epidermal growth factor (urogastrone) page 59, column 2, abstract no. 48 309e
CHEMICAL ABSTRACTS, vol. 90, no. 19, May 7, 1979, Columbus, Ohio, USA; R.L. LADDA et al. "Radioreceptor assay for epidermal growth factor", p. 260, column 2, abstract no. 148024q
CHEMICAL ABSTRACTS, vol. 88, no. 9, Feb 27, 1978, Columbus, Ohio, USA; R.H.STARKEY et al. "Radioimmunoassay of human epidermal growth factor" page 174, column 1, abstract no. 59 868j
CHEMICAL ABSTRACTS, vol. 89, no. 7, Aug 14, 1978, Columbus, Ohio, USA; G.E.DAILEY et al. "Homologous radioimmunoassay for human epidermal growth factor

(73) Proprietor: **NIHON CHEMICAL RESEARCH KABUSHIKI KAISHA also known as JAPAN CHEMICAL RESEARCH CO., LTD**, 4-20, Mikagehommachi 3-chome Higashinada-ku, Kobe Hyogo 658(JP)

(72) Inventor: **Hayashi, Kyozo**, 206 Howaito Kopo Fukagai, 828-1, Iwasaki Gifu Gifu 502(JP)
Inventor: **Kurobe, Masayuki**, 5-16, Kamitsuchii 2-chome, Gifu Gifu 502(JP)
Inventor: **Nishimuro, Satoshi**, 10-107, **Uzumoridai 2-chome Higashinada-ku, Kobe Hyogo 658(JP)**
Inventor: **Hiratani, Hajime**, 705-3, Tottori Hannan-cho, Sennan-gun Osaka 599-02(JP)

(74) Representative: **Burford, Anthony Frederick et al**, W.H. Beck, Greener & Co. 7 Stone Buildings Lincoln's Inn, London WC2A 3SZ(GB)

(56) References cited: (continuation)

(urogastrone)" page 258, column 1, abstract no. 55 933z
CHEMICAL ABSTRACTS, vol. 91, no. 9, Aug 27, 1979, Columbus, Ohio,USA; H.GREGORY et al. "Urogastrone-epidermal growth factor" page 289, column 2, abstract no. 71 082t

## Description

The present invention relates to an enzyme immunoassay method for human epidermal growth factor.

Human epidermal growth factor (hereinafter abbreviated as hEGF) is found not only in human urine and blood but also in such body fluids as saliva, gastric juice, pancreatic juice and bone marrow fluid. The urinary excretion and blood level of hEGF are known to increase in such disease as hyperthyroidism, acromegaly and Cushing's syndrome and decrease in chronic cirrhosis and gastric ulcer. It is also known that the urinary excretion of hEGF increases in certain types of malignant tumor (Hirata et al., Folia Endocrinologica Japonica, 59, 1930-1934, 1983; Hirata, Y et al., Horm. Metab. Res., 15, 261-162, 1983). To know the hEGF amounts in the body fluids of patients with these diseases provides clinically useful information.

Method of assaying hEGF in human body fluids are so far known, namely radioreceptor assay and radioimmunoassay, which use radioactive compounds, and enzyme immunoassay using beta-galactosidase-labelled antibodies, which has been established by the present inventors (Hayashi et al., Biochemistry International, 10, 859, 1985).

However, these assay methods are all unsatisfactory with regard to detection sensitivity and, in assaying samples having low hEGF contents, require procedures for the concentration of hEGF and the removal of interfering substances. In particular the assay methods using radiolabelled compounds are not suited for routine test purposes because of installation problems.

The enzyme immunoassay established by the present inventors, which uses beta-galactosidase-labelled antibodies, encounters no problems in assaying urine samples, which have high hEGF contents, since the detection limit is 0.2 ng/ml. However, other body fluids than urine all have very low hEGF contents, so that they cannot be assayed as they are. Furthermore, said assay method is disadvantageous in that in assaying hEGF in blood samples, the assay is disturbed by galactosidase-like enzymes occuring in the blood.

Accordingly, the present inventors studied intensively in an attempt to overcome the problems which may be encountered in the enzyme immunoassay using beta-galactosidase labelled antibodies and, as a result, found that the hEGF detection sensitivity of the enzyme immunoassay using antibodies coupled to beta-galactosidase largely depends on the kind of the enzyme used for antibody labelling and, surprisingly, further found that the assay sensitivity for hEGF is 100–500 times increased when antibodies coupled to peroxidase are used.

The present invention is based on the above new findings and provides an enzyme immunoassay method for epidermal growth factor in a fluid sample, which comprises reacting immobilized anti-human epidermal growth factor antibody with the fluid sample and then with peroxidase-labelled anti-human growth factor antibody, separating the solid phase from the liquid phase, and assaying the enzyme either in the solid phase or in the liquid phase so separated.

The gist of the invention lies in first causing binding of hEGF in samples to anti-hEGF antibody coupled to an insoluble carrier in the manner of antigen-antibody reaction, further causing binding of the reaction product to an anti-hEGF antibody-coupled per oxidase and assaying the activity of the bound or unbound peroxidase to thereby determine the hEGF amounts in the samples.

According to present invention, samples low in hEGF content can be assayed without troublesome procedure for concentration or removal of interfering substance. Furthermore, when a substrate capable of developing color in the visible region, such as ortho-phenylenediamine, is used as the substrate for determining enzyme activity, hEGF can be assayed with the naked eye without using any expensive apparatus.

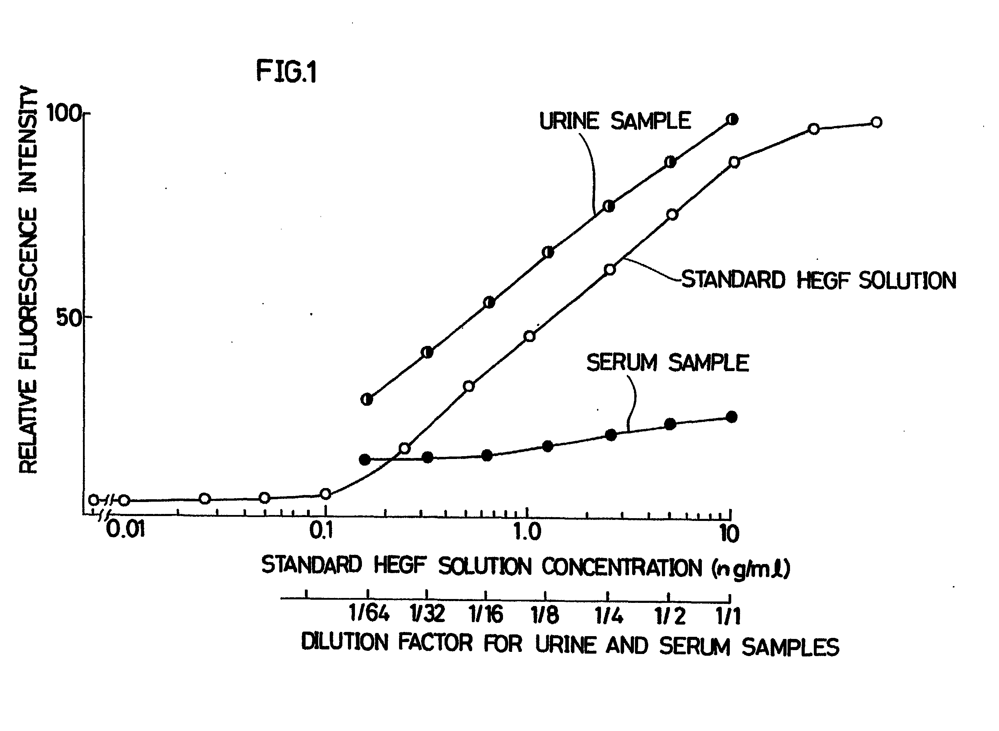

Referring to the drawings, Fig. 1, Fig. 2 and Fig. 3 each shows the hEGF assay results obtained in Reference Example 1, Example 1 and Example 2, respectively.

The anti-hEGF antibody can be obtained, for example, in the following manner: A warm-blooded animal other than human, which is capable of antibody production, for example guinea pig, rabbit, rat, mouse or goat, is immunized with hEGF as antigen by a conventional method, the blood is collected, the antiserum is separated and the antibody is purified. For the antibody purification, conventional purification means can be used. Thus, for example, the antiserum is separated by fractionation with ammonium sulfate and then purified and collected by ion exchange or gel filtration. Protein A affinity chromatography by which the antibody is adsorbed specifically may also be used. A further possible method of anti-hEGF antibody preparation comprises causing fusion between spleen cells from a warm-blooded, hEGF-immunized animal other than human and myeloma cells, picking out anti-hEGF antibody-producing fused cells and recovering monoclonal anti-hEGF antibodies. In monoclonal anti-hEGF antibody production, the mouse is used in particular. Thus, for example, the spleen is excised from an hEGF-immunized BALB/C mouse and a simple splenocyte suspension is prepared in an appropriate medium. These splenocytes are then fused with mouse-derived myeloma cells, for example SP2/0, at 37°C in a culture medium, for example RPMI1640, containing 40-50% of polyethylene glycol and then fused cells are selectively cultivated in a culture medium containing hypoxanthine, aminopteridine, thymidine and fetal calf serum. Further cloning by the limiting dilution method using thymocytes of BALB/C as feeder cells leads to separation of anti-hEGF antibody-producing cells. These cells are further cultivated in an appropriate medium, for example in RSMI1640 medium containing fetal calf serum and a monoclonal anti-hEGF antibody is recovered from the culture broth by fractionation with ammonium sulfate, ion exchange, gel filtration or affinity chromatography. It is also possible to obtain a monoclonal anti-hEGF antibody from the ascitic fluid obtained by transplanting anti-hEGF antibody-producing fused cells into the peritoneal cavity of a histocompatible animal or a nude mouse.

These antibodies may be used not only in the form of immunoglobulins but also in a fragment form, for example in the form of F(ab')₂ obtainable by antibody treatment with papain or in the form of Fab' obtainable by further reduction or in the form of F(ab)₂ obtainable of antibody treatment with pepsin.

Furthermore, these antibodies can be used in the form of immobilized antibodies attached to insoluble carriers or in the form of labelled antibodies coupled to peroxidase. Examples of usable insoluble carriers serving for antibody immobilization are polyethylene, glass, polyacrylamide, cellulose and dextrin. The anti-hEGF antibodies are physically or chemically bound to the surface of such carriers. hEGF in samples is adsorbed on these carriers in the manner of antigen-antibody reaction. Polystyrene is a particularly preferable antibody-binding carrier since it has high antibody-adsorbing capacity.

In enzyme-labelled antibody preparation, peroxidase is used as the enzyme. In peroxidase-antibody coupling, an appropriate spacer or functional group may be introduced into the enzyme in advance. It is also possible to introduce one or more functional groups into anti-hEGF antibody instead of introduction into the enzyme. The peroxidase-labelled antibody can be prepared by using a crosslinking reagent capable of binding the enzyme and antibody together making the best of the amino, hydroxyl and/or carboxyl group occurring therein and/or the functional group or groups introduced. Examples are dialdehyde compounds, dicarboxylic acid compounds, diisocyanate compounds, maleimide carboxylic acid compounds, dimaleimide compounds, thicarboxylic acid compounds, and reactive derivatives of these. An appropriate one can be selected depending on the functional groups participating in the enzyme-antibody coupling.

In the practice of the invention, 50-1,000 µl of a fluid sample is first reacted with immobilized hEGF antibody in 50-5,000 µl of a buffer solution for 12-24 hours. Then an excess of enzyme- labelled antibody is added and reaction is allowed to proceed for 1-12 hours. Thereafter, the solid phase, namely immobilized hEGF antibody-hEGF-enzyme-labeled antibody complex, is separated from the liquid phase, and one of the phases is assayed for enzyme activity. The enzyme activity determination may be carried out by an appropriate known method based on the properties of the oxidoreductase. For example, the component consumed or formed by the enzymatic reaction, such as oxygen or hydrogen peroxide, may be quantitatively determined electrochemically by means of an electrode, such as an oxygen electrode or a hydrogen peroxide electrode. Furthermore, hydrogen peroxide can be assayed by colorimetry or fluorometry using a color reagent system for hydrogen peroxide, such as 4-aminoantipyrin and phenol or dimethylaniline, or 3-methyl-2-benzothiazoline and dimethylaniline or ortho-phenylenediamine, or a fluorescence reagent such as p-hydroxyphenylacetic acid or 3-(p-hydroxyphenyl)propionic acid. Particularly the use of color reagents is suited for routine test purposes since no expensive apparatus is required and evaluation can be made by the naked eye.

The following reference examples and working examples are further illustrative of the invention.

## Reference Example 1

### (1) Preparation of anti-hEGF antiserum

hEGF was prepared from human urine by the method described in Japanese Kokai Tokkyo Koho 58-219124 and U. S. Patent 4,528,186. hEGF (0.5 mg) was dissolved in 0.5 ml of physiological saline, an equal volume of Freund's complete adjuvant and, after mixing for emulsification, the mixture emulsion was injected into a rabbit at several sites on the back, followed by three subcutaneous injections of the same emulsion in the same dose at 2-week intervals. Ten days after the final immunization, the whole blood was collected, allowed to stand at room temperature for coagulation and then centrifuged at 3,000 rpm for 10 minutes to give anti-hEGF-containing antiserum.

### (2) Preparation of microtiter with antibody immobilized thereon

The anti-hEGF antibody-containing antiserum obtained in (1) was applied to a column packed with Sepharose 4B carrying protein A immobilized thereon to thereby cause γ-globulin to be adsorbed thereon. Then the column was washed well with 0.1 M phosphate buffer containing 0.15 M NaCl (pH 8.0) and then γ-globulin was eluted from the column with 1 M acetic acid. The eluate was purified by dialyzing against 0.05 M Tris hydrochloride buffer (pH 8.5). The thus-purified anti-hEGF antibody solution was adjusted to a concentration of 200 µg per milliliter and distributed in 150-µl portions into the wells of a 96-well polystyrene microplate (for EIA; product of Nunc, Denmark), and the antibody was immobilized by incubation at 37°C for 30 minutes The plate was washed well with 0.1 M phosphate buffer (pH 7.0) containing 0.1 M NaCl, 0.1% bovine albumin, 0.1% NaN₃ and 1 mM MgCl₂ to give a plate with anti-hEGF antibody immobilized thereon.

### (3) Preparation of beta-galactosidase-coupled anti-hEGF antibody

A 10-mg portion of the purified anti-hEGF antibody prepared in (2) was digested with pepsin (Boehringer Mannheim-Yamanouchi, Japan) in 0.1 M acetate buffer (pH 4.5). The digest was purified on a Sephadex G-100 (Pharmacia Japan) column and then deprived of undigested antibody by passing a Sepharose column with protein A immobilized thereon. The thus-obtained F(ab')₂ fragment was reduced with 2-mercaptoethanolamine and purified on a Sephadex G-25 (Pharmacia) column to give an Fab' fragment solution A saturated N,N'-o-phenylenedimaleimide solution (1 ml) was added to 1 ml of the Fab' fragment solution, the mixture was incubated at 30°C for 15 minutes and immediately thereafter applied to a Sephadex G-25 column, and the initial eluate was collected and concentrated to I ml. To the concentrate was added 0.5 mg of beta-D-galactosidase (β-D-galactoside hydrolase, EC 3.2.1.23; Boehringer Mannheim-Yamanouchi, Japan). After incubation at 30°C for 20 minutes, the reaction mixture was made neutral by addition of I N NaOH. After further addition of bovine serum albumin, the mixture was allowed to stand overnight. The reaction mixture was purified on Sepharose CL-6B (Pharmacia Japan) equibrated with 0.1 M phosphate buffer (pH 7.0) containing 0.3 M NaCl, 0.1% bovine serum albumin, 0.1% NaN₃ and 1 mM MgCl₂. Thus was obtained Fab'-beta-D-galactosidase complex.

(4) Assay of hEGF

Purified hEGF was dissolved in a series of concentrations within the range of 0.01 ng to 50 ng per milliliter in 0.1 M phosphate buffer (pH 7.0) containing 0.1 M NaCl, 0.1% bovine serum albumin, 0.1% NaN₃ and 1 mM NaCl₂ and the solutions were used as standard solutions. A 150-μl portion of each standard hEGF solution was added to a well of the microplate with anti-hEGF immobilized thereon as prepared in (2). After overnight standing at room temperature, each well was washed with a buffer, 0.2 milliunit of beta-galactosidase-coupled antibody in solution was added to each well and the reaction was allowed to proceed overnight at room temperature. Each well was washed with a buffer, 150 μl of 0.06 mM 4-methylumbelliferylβ-D-galactoside solution containing 0.1% Triton X-100 was added to each well and the reaction was allowed to proceed at room temperature for 20 minutes. Then, 50 μl of 0.1 M glycine-NaOH solution (pH 10.3) was added to each well and the resultant fluorescence was measured on a fluorometer (Hitachi model 650-60) at the excitation wavelength of 360 nm and the measurement wavelength of 450 nm. As the results shown in Fig. 1 indicate, the detection limit was 0.2 ng/ml. Assay of hEGF in human urine and serum was attempted using this assay system. While hEGF in urine could be assayed easily up to 29 ng/ml, hEGF in serum could not be assayed exactly due to lack of parallelism between the actual assay curve and the standard hEGF assay curve.

Example 1

(1) Preparation of anti-hEGF antiserum

The antiserum described in Reference Example 1-(1) was used.

(2) Preparation of microplate with antibody immobilized thereon

The plate described in Reference Example 1-(2) was used.

(3) Preparation of peroxidase-coupled anti-hEGF antibody

Peroxidase (2 mg; special reagent for enzyme immunoassay; Nordic, Netherlands) was dissolved in 0.3 ml of 0.1 M phosphate buffer (pH 6.0). To the solution was added a 20-μl N,N-dimethylformamide solution of 1.6 mg of N-succinimidyl 4-(N-maleimidomethyl)cyclohexane-1-carboxylate (Wako Pure Chemical Industries). The mixture was incubated at 60°C for 30 minutes. After removal of the resultant precipitate by centrifugation, the reaction mixture was subjected to gel filtration using a Sephadex G-25 column equilibrated with 0.1 M phosphate bffer (pH 6.0) to give a maleimidated peroxidase solution. Thereto was added 2.86 mg of the Fab' fragment of anti-hEGF antibody as described in Reference Example 1-(3). After 30 minutes of reaction at 4°C, the reaction mixture was purified on a column packed with Ultrogel Ac44 (Pharmacia) equilibrated with 0.1 M phosphate buffer (pH 6.0). Thus was obtained peroxidase-coupled Fab' fragment.

(4) Assay of hEGF

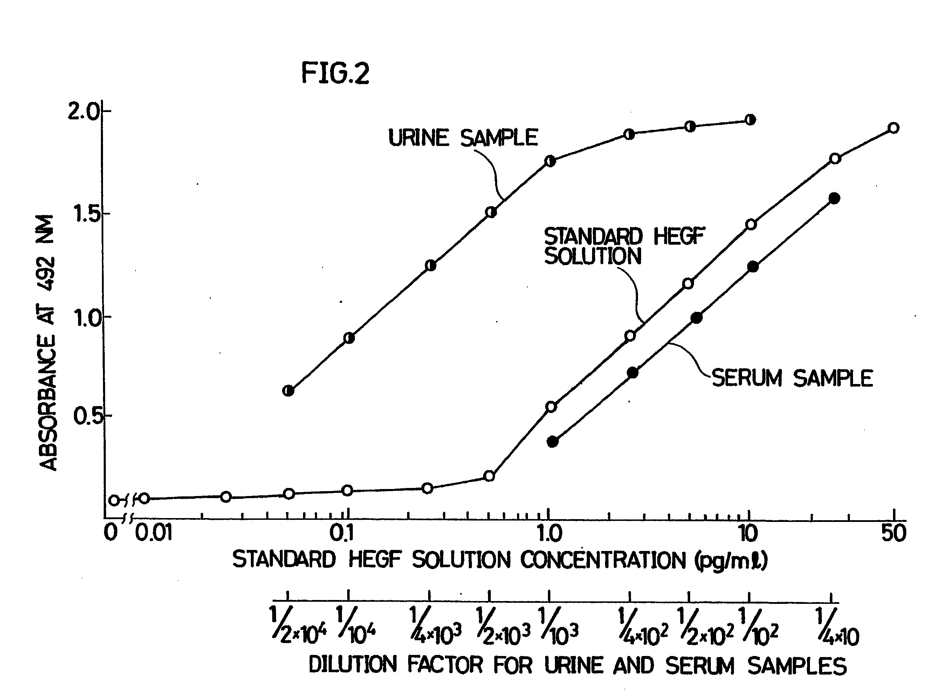

Purified hEGF was dissolved in concentrations of 0.01 pg to 50 pg per milliliter in 0.1 M phosphate buffer (pH 7.0) containing 0.1 M NaCl, 0.1% bovine serum albumin and 1 mM MgCl₂. The solutions thus prepared were used as standard hEGF, solutions. A 150-μl portion of each standard solution was added to a well of the microplate with anti-hEGF antibody immobilized thereon as prepared in (2). After standing overnight at room temperature, each well was washed with a buffer, 150 μl of a buffer containing 4.3 ng of the peroxidase-coupled Fab' fragment prepared in (3) was added and the reaction was allowed to proceed at room temperature for 6 hours. Again, each well was washed well with a buffer. Thereafter, 150 μl of 0.1 M citrate buffer (pH 5.0) containing 400 μg/ml of o-phenylenediamine and 0.01% hydrogen peroxide was added to each well and the reaction was allowed to proceed at 25°C for 1 hour, followed by addition of 25 μl of 8 N sulfuric acid and absorbance measurement at 492 nm on a multiscanning photometer (Titertek; Flow, USA). The results thus obtained are Shown in Fig. 2. The detection limit was 0.5 pg/ml. Assay of hEGF in human urine and serum samples was attempted using this assay system. The actual assay curves for these samples were in parallel with the standard hEGF assay curve. The hEGF contents in the urine and serum samples were 25 ng/ml and 580 pg/ml, respectively.

Example 2

(1) Preparation of anti-hEGF antiserum

The antiserum described in Reference Example 1-(1) was used.

(2) Preparation of antibody immobilized on polystyrene beads

Polystyrene beads (3.2 mm in diameter; Immunobeads; Bio-Rad, Japan) were immersed in a 0.2 mg/ml solution of the purified anti-hEGF IgG described in Reference Example 1-(2) in 0.05 M Tris-hydrochloride buffer (pH 8.5). After allowing to stand at room temperature for 30 minutes, the beads were taken out and washed with 0.1 M phosphate buffer (pH 7.0) and then with 0.1 M phosphate buffer (pH 7.0) containing 0.3 M NaCl, 0.1% bovine serum albumin, 0.1% NaN$_3$ and 1 mM MgCl$_2$. Thus were obtained beads with antibody immobilized thereon.

### (3) Preparation of peroxidase-coupled antibody

The product described in Example 1-(3) was used.

### (4) Assay of hEGF

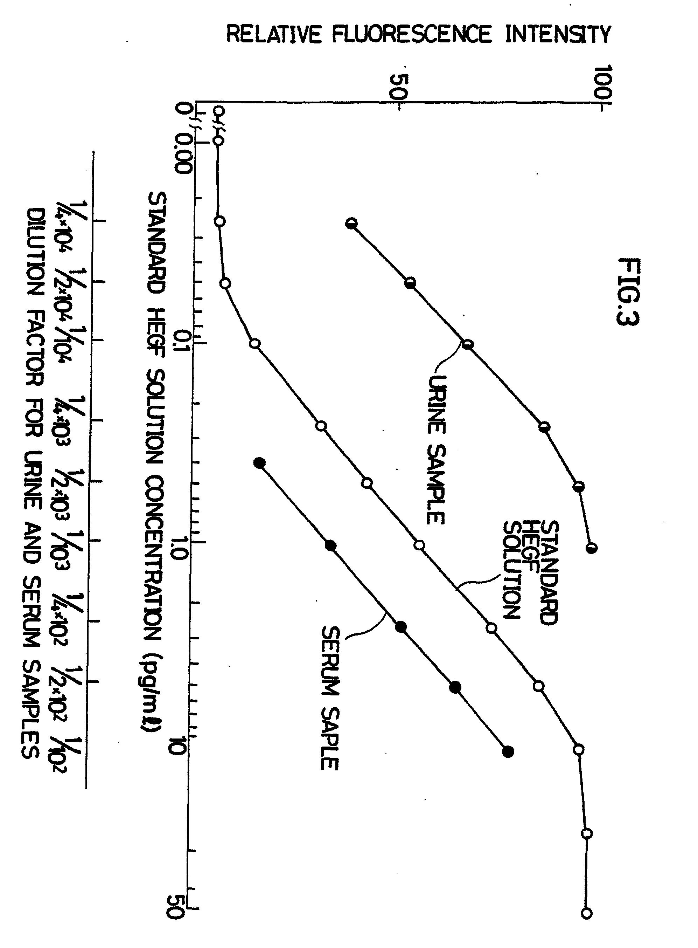

Standard hEGF solutions were prepared by dissolving purified hEGF, in concentrations of 0.01 pg to 50 pg per milliliter, in 0.1 M phosphate buffer (pH 7) containing 0.1 M NaCl, 0.1% bovine serum albumin and 1 mM MgCl$_2$. Each standard hEGF solution (100 μl) was added to a glass test tube (1.0 x 10.0 cm) containing one of the beads with antibody immobilized thereon as prepared in (2). After overnight standing at 4°C, the beads were washed with 0.1 M phosphate buffer (pH 7.0) containing 0.1% bovine serum albumin. Then, a buffer solution (200 μl) containing 4.3 ng of the peroxidase-coupled Fab′ fragment prepared in (3) was added to each test tube. The tube was shaken gently at room temperature for 6 hours and then the bead was washed well with a buffer and transferred to another test tube. Thereto was added 100 μl of 0.6% (w/v) 3-(para-hydroxyphenyl)propionic acid (Wako Pure Chemical Industries). Five minutes later, 100 μl of 0.015% aqueous hydrogen peroxide was added and the mixture was incubated at 25°C for 1 hour. The reaction was then terminated by addition of 200 μl of 0.1 M glycine-NaOH buffer (pH 10.3) and the fluorescence intensity was measured on a fluorometer (Hitachi model 650-60) at the excitation wavelength of 320 nm and the measurement wavelength of 405 nm. The results thus obtained are shown in Fig. 3. The detection limit was 0.1 pg/ml. Assay of hEGF in human urine and serum samples was attempted using this assay system. The actual assay curves for these samples were in parallel with the standard hEGF assay curve and the hEGF contents in the urine and serum samples were found to be 17 ng/ml and 323 pg/ml, respectively.

### Claims

1. An enzyme immunoassay method for epidermal growth factor in a fluid sample, which comprises reacting immobilized anti-human epidermal growth factor antibody with the fluid sample and then with an enzyme-labelled anti-human epidermal growth factor antibody, separating the resultant solid phase from the liquid phase, and assaying the enzyme either in the solid phase or in the liquid phase so separated, characterised in that the said enzyme-labelled antibody is peroxidase-labelled anti-human epidermal growth factor antibody.

2. A method as claimed in Claim 1, wherein the epidermal growth factor is human epidermal growth factor.

3. A method as claimed in Claim 1 or Claim 2, wherein the anti-epidermal growth factor is used in the form of immuno-globulins, or in a fragment form of F(ab′)$_2$, FAB′ or F(ab)$_2$.

4. A method as claimed in any one of the preceding claims, wherein the immobilized anti-epidermal growth factor antibody is anti-epidermal growth factor antibody coupled to an insolubilizing carrier.

5. A method as claimed in any one of the preceding claims, wherein the anti-epidermal growth factor antibody is an antibody obtained by immunization of a warm-blooded animal.

6. A method as claimed in any one of Claims 1 to 5, wherein the anti-epidermal growth factor antibody is a monoclonal antibody.

7. The use of a peroxidase-labelled anti-human epidermal growth factor antibody in an immunoassay for epidermal growth factor.

8. Peroxidase-labelled anti-human epidermal growth factor antibody.

### Patentansprüche

1. Enzym-Immunoassay-Verfahren für einen epidermalen Wachstumsfaktor in einer flüssigen Probe, beidem man einen immobilisierten anti-humanen epidermalen Wachstumsfaktor-Antikörper mit der flüssigen Probe und anschließend mit einem Enzymmarkierten anti-humanen epidermalen Wachstumsfaktor-Antikörper zur Reaktion bringt, die entstandene feste Phase von der flüssigen Phase trennt und das Enzym entweder in der abgetrennten festen oder in der abgetrennten flüssigen Phase nachweist, dadurch gekennzeichnet, daß der Enzym-markierte Antikörper ein mit Peroxidase markierter anti-humaner epidermaler Wachstumsfaktor-Antikörper ist.

2. Verfahren nach Anspruch 1, dadurch gekennzeichnet, daß der epidermale Wachstumsfaktor ein menschlicher epidermaler Wachstumsfaktor ist.

3. Verfahren nach Anspruch 1 oder 2, dadurch gekennzeichnet, daß der anti-epidermale Wachstumsfaktor in Form eines Immunglobulins oder in Form eines Fragments von F(ab′)$_2$, FAB′ oder F(ab)$_2$ verwendet wird.

4. Verfahren nach einem der vorhergehenden Ansprüche, dadurch gekennzeichnet, daß der immobilisierte anti-epidermale Wachstumsfaktor-Antikörper ein an einen unlöslich machenden Träger gekoppelter anti-epidermaler Wachstumsfaktor-Antikörper ist.

5. Verfahren nach einem der vorhergehenden Ansprüche, dadurch gekennzeichnet, daß der anti-epidermale Wachstumsfaktor-Antikörper ein durch Immunisierung eines warmblütigen Tieres gewonnener Antikörper ist.

6. Verfahren nach einem der Ansprüche 1 bis 5, dadurch gekennzeichnet, daß der anti-epidermale Wachstumsfaktor-Antikörper ein monoklonaler Antikörper ist.

7. Verwendung eines Peroxidase-markierten anti-humanen epidermalen Wachstumsfaktor-Antikörpers in einem Immunoassay für einen epidermalen Wachstumsfaktor.

8. Peroxidase-markierter anti-humaner epidermaler Wachstumsfaktor-Antikörper.

## Revendications

1. Procédé d'immunodosage enzymatique du facteur de croissance épidermique, qui consiste à faire réagir de l'anticorps anti-(facteur de croissance épidermique humain) immobilisé avec l'échantillon de fluide puis avec un anticorps anti-(facteur de croissance épidermique humain) marqué par une enzyme, à séparer la phase solide résultante de la phase liquide, et à doser l'enzyme soit dans la phase solide soit dans la phase liquide ainsi séparées, caractérisé en ce que ledit anticorps marqué par une enzyme est l'anticorps anti-(facteur de croissance épidermique humain) marqué à la peroxydase.

2. Procédé selon la revendication 1, dans lequel le facteur de croissance épidermique est le facteur de croissance épidermique humain.

3. Procédé selon la revendication 1 ou 2, dans lequel l'anticorps anti-(facteur de croissance épidermique) est utilisé sous la forme d'immunoglobulines, ou sous la forme fragmentée $F(ab')_2$, $Fab'$ ou $F(ab)_2$.

4. Procédé selon l'une quelconque des revendications précédentes, dans lequel l'anticorps anti-(facteur de croissance épidermique) immobilisé est un anticorps anti-(facteur de croissance épidermique) couplé à un support insoluble.

5. Procédé selon l'une quelconque des revendications précédentes, dans lequel l'anticorps anti-(facteur de croissance épidermique) est un anticorps obtenu par immunisation d'un animal à sang chaud.

6. Procédé selon l'une quelconque des revendications 1 à 5, dans lequel l'anticorps anti-(facteur de croissance épidermique) est un anticorps monoclonal.

7. Utilisation d'un anticorps anti-(facteur de croissance épidermique humain) marqué à la peroxydase dans un immunodosage du facteur de croissance épidermique.

8. Anticorps anti-(facteur de croissance épidermique humain) marqué à la peroxydase.

FIG.1

EP 0 228 810 B1

FIG.2

RELATIVE FLUORESCENCE INTENSITY

FIG.3

URINE SAMPLE

STANDARD HEGF SOLUTION

SERUM SAPLE

STANDARD HEGF SOLUTION CONCENTRATION (pg/ml)

DILUTION FACTOR FOR URINE AND SERUM SAMPLES